# FASCICULE DE BREVET EUROPEEN

(11) **EP 0 558 623 B1**
(45) Date de publication et mention de la délivrance du brevet: **21.09.1994**
(21) Numéro de dépôt: 92900928.0
(22) Date de dépôt: 22.11.1991
(51) Int. Cl.: C07D 305/14, A61K 31/335, C07D 263/04

(54) **PROCEDE DE PREPARATION DE DERIVES DU TAXANE, NOUVEAUX DERIVES OBTENUS ET COMPOSITIONS PHARMACEUTIQUES QUI LES CONTIENNENT**
VERFAHREN ZUR HERSTELLUNG VON TAXANDERIVATEN, SO ERHALTENE NEUE DERIVATE UND PHARMAZEUTISCHE ZUSAMMENSETZUNGEN, DIE SIE ENTHALTEN
METHOD FOR PREPARING TAXANE DERIVATIVES, NOVEL DERIVATIVES THEREBY OBTAINED AND PHARMACEUTICAL COMPOSITIONS CONTAINING SAME

(30) Priorité: 23.11.1990 FR 9014635; 25.07.1991 FR 9109423
(43) Date de publication de la demande: 08.09.1993
(60) Demande divisionnaire: 93119434.4
(73) Titulaire: RHONE-POULENC RORER S.A., 92160 Antony (FR)
(72) Inventeur: BOURZAT, Jean-Dominique, F-94300 Vincennes (FR); COMMERCON, Alain, F-94400 Vitry-sur-Seine (FR); PARIS, Jean-Marc, F-77360 Vaires-sur-Marne (FR)
(74) Mandataire: Pilard, Jacques
(86) Numéro de dépôt international: FR9100928
(87) Numéro de publication internationale: WO9209589

(56) Documents cités:
- EP-A- 0 253 739
- EP-A- 0 336 841
- J Med Chem 1991, vol 34, No.3, pp 992-998
- Chemical Abstracts, vol. 109, No.9, 29 August 1988 (Columbus, Ohio, US) N F Magri et al., page 13, abstract 66329q
- Chemical Abstracts, vol.114, No.11, 1991 (Columbus, Ohio, US) B Monsarrat et al., page 13, abstract 94569q

## Description

La présente invention concerne un procédé de préparation de dérivés du taxane de formule générale : 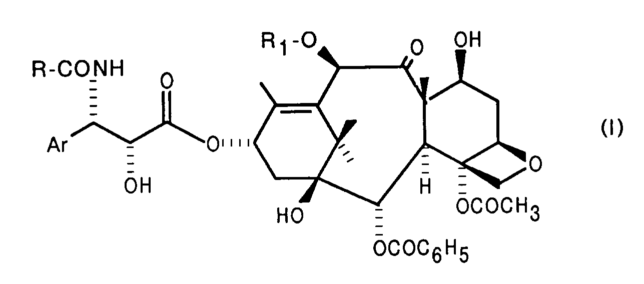
dans laquelle R représente un radical t.butoxy ou phényle, R₁ représente un atome d'hydrogène et Ar représente un radical phényle substitué ou α- ou β-naphtyle éventuellement substitué par un ou plusieurs atomes ou radicaux choisis parmi les atomes d'halogène (fluor, chlore, brome, iode) et les radicaux alkyles, aryles, arylalcoyles, alkoxy, alkylthio, aryloxy, arylthio, mercapto, acylamino, aroylamino, alkoxycarbonylamino, amino, alkylamino, dialkylamino, carboxy, alkoxycarbonyle, carbamoyle, dialkylcarbamoyle, cyano, nitro et trifluorométhyle, étant entendu que les radicaux alkyles et les portions alkyles des autres radicaux contiennent 1 à 4 atomes de carbone et que les radicaux aryles sont des radicaux phényles ou α- ou β-naphtyles.

La présente invention concerne également les nouveaux dérivés du taxane de formule générale (I).

Plus particulièrement l'invention concerne les nouveaux dérivés du taxane de formule générale (I) dans laquelle Ar représente un radical phényle substitué par un ou plusieurs atomes ou radicaux, identiques ou différents, choisis parmi les atomes d'halogène et les radicaux alkyles, alkoxy, amino, alkylamino, dialkylamino, acylamino, alkoxycarbonylamino et trifluorométhyle.

Plus particulièrement encore, l'invention concerne les produits de formule générale (I) dans laquelle Ar représente un radical phényle substitué par un atome de chlore ou de fluor, ou par un radical alkyle (méthyle), alkoxy (méthoxy), dialkylamino (diméthylamino), acylamino (acétylamino) ou alkoxycarbonylamino (t.butoxycarbonylamino).

Dans EP-A-0 253 739 est décrite la préparation du taxol à partir du Taxotère (t.butoxycarbonylamino-3 phényl-3 hydroxy-2 propionate-(2R,3S) d'acétoxy-4 benzoyloxy-2 époxy-5β,20 dihydroxy-7β,10β oxo-9 taxène-11 yle-13α) qui peut lui-même être obtenu selon le procédé décrit dans EP-A-0 336 841 par condensation de l'acide t.butoxycarbonylamino-3 phényl-3 hydroxy (protégé)-2 sur la désacétyl-10 baccatine III convenablement protégée. Le taxol et le Taxotère présentent des propriétés antitumorales et antileucémiques. D'après Chem. Abstr. 114 94569 q (1991), les métabolites du taxol qui sont des dérivés hydroxylés sur le noyau phényle en -3' ou sur le noyau phényle du benzoyle en -2 sont nettement moins actifs que le taxol.

Selon l'invention, les dérivés du taxane de formule générale (I) peuvent être obtenus de la manière suivante :
1) le dérivé de l'oxazolidine de formule générale : 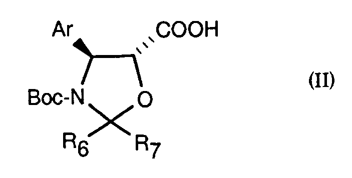 dans laquelle Ar est défini comme précédemment, Boc représente le radical t.butoxycarbonyle et R₆ et R₇, identiques ou différents, représentent un radical alkyle contenant 1 à 4 atomes de carbone éventullement substitué par un ou plusieurs radicaux aryles (phényle), ou aryle (phényle), ou bien R₆ et R₇ forment ensemble avec l'atome de carbone auquel ils sont liés un cycle ayant de 4 à 7 chaînons, est condensé avec un dérivé du taxane de formule générale : 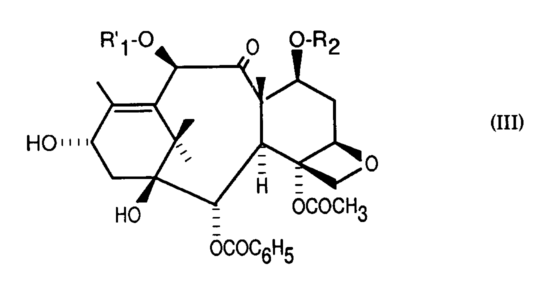 dans laquelle R'₁ représente un groupement protecteur de la fonction hydroxy tel que le radical trichloro-2,2,2 éthoxycarbonyle et R₂ représente un groupement protecteur de la fonction hydroxy tel que le radical trichloro-2,2,2 éthoxycarbonyle ou un radical trialcoylsilyle dont chaque partie alkyle contient 1 à 4 atomes de carbone pour donner le produit de formule générale : 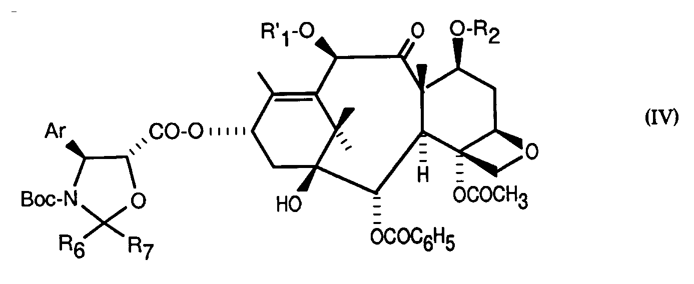 dans laquelle Ar, R'₁, R₂, R₆ et R₇ sont définis comme précédemment.
   Généralement, l'estérification est effectuée en présence d'un agent de condensation tel qu'un carbodiimide comme le dicyclohexylcarbdiimide ou un carbonate réactif comme le dipyridyl-2 carbonate et d'un agent d'activation tel qu'une dialkylaminopyridine comme la diméthylamino-4 pyridine en opérant dans un solvant organique tel que le benzène, le toluène, les xylènes, l'éthylbenzène, l'isopropylbenzène ou le chlorobenzène à une température comprise entre 60 et 90°C.
   Il est particulièrement avantageux d'utiliser un excès molaire d'acide de formule (II) par rapport au dérivé du taxane de formule (III), l'agent de condensation étant utilisé en quantité stoechiométrique par rapport à l'acide de formule (II) et l'agent d'activation étant utilisé en quantité stoechiométrique par rapport au dérivé du taxane de formule (III).
2) le produit de formule générale (IV) dans laquelle R₂ représente un radical trichloro-2,2,2 éthoxycarbonyle est traité par un acide minéral ou organique éventuellement dans un alcool de façon à obtenir le produit de formule générale : 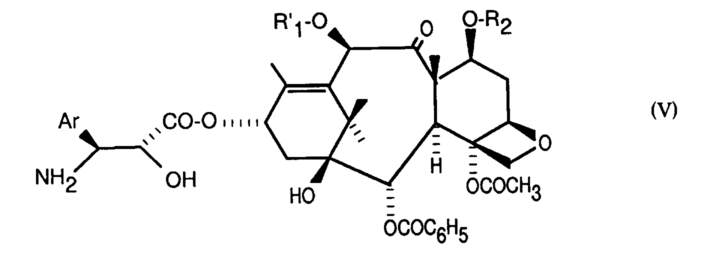 dans laquelle Ar et R'₁ sont définis comme précédemment et R₂ représente un radical trichloro-2,2,2 éthoxycarbonyle.
   Généralement, on utilise l'acide formique éventuellement dans un alcool tel que l'éthanol ou l'acide chlorhydrique gazeux dans un alcool tel que l'éthanol,
3) le produit de formule générale (V) est traité par un composé qui permet d'introduire, sur la fonction amino, un radical t.butoxycarbonyle ou benzoyle pour obtenir le produit de formule : 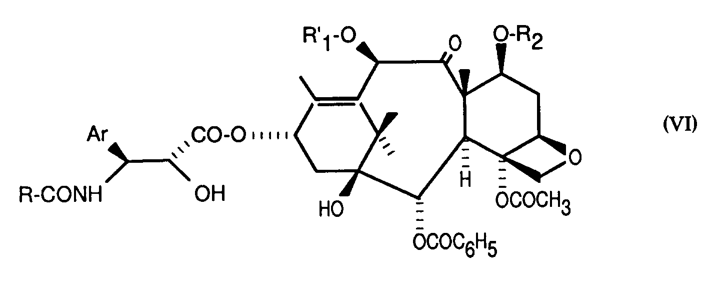 dans laquelle Ar, R et R'₁ sont définis comme précédemment et R₂ représente un radical trichloro-2,2,2 éthoxycarbonyle.
   Généralement on fait réagir le dicarbonate de di-tert.butyle ou le chlorure de benzoyle sur le produit de formule générale (VI) en opérant dans un solvant organique tel que le chlorure de méthylène en présence d'une base minérale telle que le bicarbonate de sodium ou d'une base organique telle qu'une amine tertiaire comme la triéthylamine,
4) le produit de formule générale (VI) est transformé en produit de formule générale (I) par remplacement des groupements trichloro-2,2,2 éthoxycarbonyle représentés par R'₁ et R₂ par des atomes d'hydrogène sans toucher au reste de la molécule.
   Généralement le produit de formule générale (VI) est traité par le zinc en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone en présence de zinc.

Le produit de formule générale (III) peut être préparé dans les conditions décrites dans le brevet européen EP 0 336 841.

L'acide de formule générale (II) peut être obtenu par saponification en milieu basique de l'ester de formule générale : 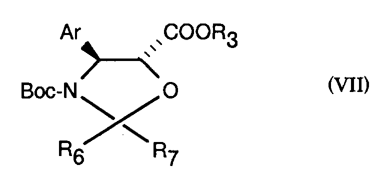
dans laquelle Ar, R₆ et R₇ sont définis comme précédemment et R₃ représente un radical alcoyle contenant 1 à 4 atomes de carbone éventuellement substitué par un radical phényle.

Généralement, la saponification est effectuée au moyen d'une base minérale telle qu'un hydroxyde de métal alcalin (lithium, sodium, potassium), un carbonate ou bicarbonate de métal alcalin (bicarbonate de sodium, carbonate ou bicarbonate de potassium) en milieu hydro-alcoolique tel qu'un mélange méthanol-eau, à une température comprise entre 10 et 40°C, de préférence voisine de 20°C.

L'ester de formule générale (VII) peut être obtenu par action d'un méthoxy alcène éventuellement substitué par un ou plusieurs radicaux aryles (méthoxy-2 propène), d'un gem-diméthoxy alcane éventuellement substitué par un ou plusieurs radicaux aryles (diméthoxy-2,2 propane) ou un gem-diméthoxycycloalcane contenant 4 à 7 atomes de carbone sur un dérivé de la phénylisosérine de formule générale : 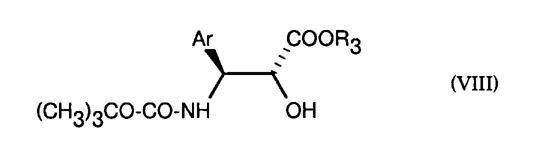
dans laquelle Ar et R₃ sont définis comme précédemment sous forme racémique ou, de préférence, sous forme 2R,3S.

Généralement, la réaction du méthoxy alcène ou du gem-diméthoxy alcane ou du gem-diméthoxycycloalcane avec le produit de formule générale (VIII) est effectuée en opérant dans un solvant organique inerte en présence d'un acide fort tel que l'acide p.toluènesulfonique à une température comprise entre 0°C et la température d'ébullition du mélange réactionnel. Les solvants qui conviennent particulièrement bien sont choisis parmi les hydrocarbures aromatiques (benzène, toluène, xylène).

Le produit de formule générale (VIII) peut être obtenu par acylation d'un dérivé de la β-phénylisosérine de formule générale : 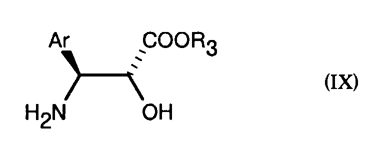
dans laquelle Ar et R₃ sont définis comme précédemment.

La réaction est généralement mise en oeuvre en faisant réagir le dicarbonate de di-tert.butyle en opérant dans un solvant organique inerte tel qu'un ester comme l'acétate de méthyle ou d'éthyle à une température comprise entre 0 et 40°C, de préférence voisine de 20°C.

Le dérivé de la β-phénylisosérine de formule générale (IX) peut être obtenu par réduction d'un hydroxy-azoture de formule générale : 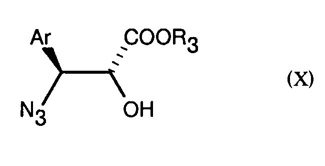
dans laquelle Ar et R₃ sont définis comme précédemment.

Généralement la réduction est effectuée au moyen d'hydrogène en présence d'un catalyseur tel que le palladium sur charbon en opérant dans un solvant organique inerte tel que l'acétate d'éthyle. On opère de préférence à une température comprise entre 0 et 50°C. Il est avantageux d'effectuer l'hydrogénation sous une pression comprise entre 1 et 5 bars.

Le produit de formule générale (X) peut être obtenu par action d'un azoture de métal alcalin tel que l'azoture de sodium sur un ester de l'acide β-phénylglycidique de formule générale : 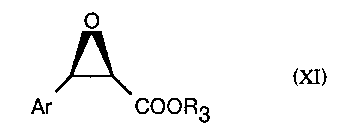
dans laquelle Ar et R₃ sont définis comme précédemment.

Généralement, on opère dans un mélange hydroorganique tel qu'un mélange eau-tétrahydrofuranne à la température de reflux du mélange réactionnel.

L'ester de formule générale (XI) peut être obtenu par déhydrohalogénation d'un produit de formule générale : 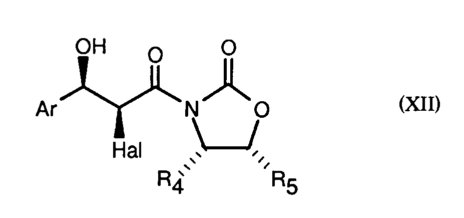
dans laquelle Ar est défini comme précédemment, Hal représente un atome d'halogène, de préférence un atome de brome et R₄ et R₅, identiques ou différents, représentent un radical alcoyle contenant 1 à 4 atomes de carbone ou phényle.

Généralement, on opère en présence d'un excès d'un alcoolate de métal alcalin, éventuellement préparé in situ, dans un solvant organique inerte tel que le tétrahydrofuranne à une température comprise entre -80°C et +25°C.

Le produit de formule générale (XII) peut être obtenu par action d'un aldéhyde de formule générale :

Ar-CHO (XIII)

dans laquelle Ar est défini comme précédemment sur un halogénure de formule générale : 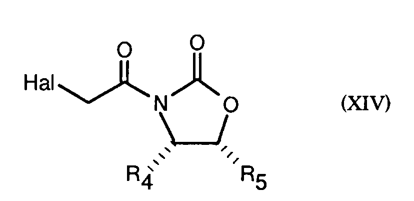
dans laquelle Hal, R₄ et R₅ sont définis comme précédemment, préalablement anionisé.

Généralement, on opère dans un solvant organique inerte choisi parmi les éthers (éther éthylique) et les hydrocarbures aliphatiques halogénés (chlorure de méthylène) à une température comprise entre -80°C et 25°C, en présence d'une amine tertiaire (triéthylamine) et d'un agent d'énolisation (triflate de di-n-butylbore).

Le produit de formule générale (XIV) peut être obtenu par action d'un halogénure d'un acide halogénoacétique, de préférence, le bromure de l'acide bromoacétique sur l'oxazolidinone correspondante.

Les spectres de résonance magnétique nucléaire du proton sont effectués dans la chloroforme deutéré. Selon la nature de la chaîne latérale, la numérotation des atomes est la suivante : 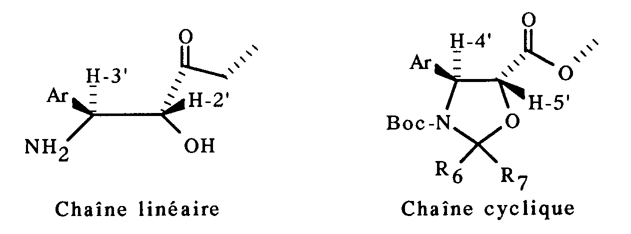
Les abréviations utilisées ont les significations suivantes :
s = singulet
d = doublet
dd = doublet de doublet
t = triplet
q = quadruplet
m = massif
Les exemples suivants illustrent la présente invention.

### EXEMPLE 1

Une solution de 0,5 g de tert-butoxycarbonylamino-3 (méthyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α dans un mélange de 10 cm3 de méthanol et de 10 cm3 d'acide acétique est chauffée sous agitation et sous atmosphère d'argon jusqu'à une température voisine de 60°C puis additionnée de 1 g de zinc en poudre. Le mélange réactionnel est ensuite agité pendant 30 minutes à 60°C puis refroidi à une température voisine de 20°C et filtré sur verre fritté garni de célite. Le verte fritté est lavé par 3 fois 10 cm3 de dichlorométhane et les filtrats sont réunis puis concentrés à sec sous pression réduite (2,7 kPa) à une température voisine de 40°C.

Le résidu est additionné de 20 cm3 d'eau distillée et le solide cristallisé est séparé par filtration, lavé par 4 fois 5 cm3 d'eau distillée et séché sous pression réduite (0,27 kPa) à 20°C pendant 16 heures. On obtient 0,25 g d'une meringue blanche que l'on purifie par chromatographie sur 40 g de silice (0,063-0,2 mm) contenus dans une colonne de 2 cm de diamètre [éluant : dichlorométhane-méthanol (97-3 en volumes)] en recueillant des fractions de 20 cm3. Les fractions 5 à 12 sont réunies et concentrées à sec sous pression réduite (0,27 kPa) à 40°C pendant 16 heures. On obtient ainsi 0,2 g de tert-butoxycarbonylamino-3 (méthyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰D = 32° (c = 0,1 ; méthanol)
- spectre de RMN (250 MHz ; CDCl₃)
   δ (ppm) : 1,14 (s, 3H : -CH₃ 16 ou 17) ; 1,24 (s, 3H : -CH₃ 16 ou 17) ; 1,35 (s, 9H : -C(CH₃)₃) ; 1,7 (s, 1H : -OH 1) ; 1,77 (s, 3H : -CH₃ 19) ; 1,85 (m, 1H : - (CH)-H 6) ; 1,87 (s, 3H : -CH₃ 18) ; 2,26 (m, 2H : -CH₂- 14) ; 2,33 (s, 3H : - COCH₃) ; 2,4 (s, 3H : CH₃-C₆H₄) ; 2,6 (ddd, 1H, J=6,5, 9,5 et 15 Hz : -(CH)-H 6) ; 3,38 (d, 1H, J=5,5 Hz : -OH 2') ; 3,92 (d, 1H, j=7 Hz :-H 3) ; 4,18 (d, 1H, J=8 Hz : -(CH)-H 20) ; 4,22 (m, 2H : -H 7 et -OH 10) ; 4,33 (d, 1H, J=8 Hz : -(CH)-H 20) ; 4,6 (m, 1H : -H 2') ; 4,96 (dd, 1H, J=1,5 et 9,5 Hz : -H 5) ; 5,22 (s, 1H : -H 10) ; 5,22 (m, 1H : -H 3') ; 5,4 (d, 1H, J=9 Hz : -NHCO-) ; 5,68 (d, 1H, J=7 Hz : - H 2) ; 6,2 (t, 1H, J=9 Hz : -H 13) ; 7,23 (AB, 4H, J_{AB}=8 Hz : CH₃-C₆H₄) ; 7,5 [t, 2H, J=7,5 Hz :-OCOC₆H₅(-H 3 et -H 5)] ; 7,62 [tt, 1H, J=1 et 7,5 Hz : - OCOC₆H₅(-H 4)] ; 8,12 [d, 2H, J=7,5 Hz : -OCOC₆H₅(-H 2 et -H 6)].

Le tert-butoxycarbonylamino-3 (méthyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α peut être préparé de la manière suivante :
A une solution de 0,45 g d'amino-3 hydroxy-2 (méthyl-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α dans 5 cm3 de dichlorométhane, maintenue sous atmosphère d'argon, on ajoute 0,037 g d'hydrogénocarbonate de sodium puis goutte à goutte, à une température voisine de 20°C, une solution de 0,108 g de dicarbonate de di.tert-butyle dans 5 cm3 de dichlorométhane. La solution obtenue est agitée pendant 24 heures à une température voisine de 20°C puis additionnée d'un mélange de 15 cm3 d'eau distillée et de 20 cm3 de dichlorométhane. La phase aqueuse est séparée par décantation puis réextraite par 20 cm3 de dichlorométhane. Les phases organiques sont réunies, séchées sur sulfate de magnésium, filtrées puis concentrées à sec sous pression réduite (2,7 kPa) à 40°C. On obtient ainsi 0,5 g de tert-butoxycarbonylamino-3 (méthyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

L'amino-3 hydroxy-2 (méthyl-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α peut être préparé de la manière suivante :
Une solution de 0,6 g de tert-butoxycarbonyl-3 diméthyl-2,2 (méthyl-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α dans 6 cm3 d'acide formique est agitée pendant 4 heures à une température voisine de 20°C puis concentrée à sec sous pression réduite (2,7 kPa) à 40°C. Le résidu est additionné de 40 cm3 de toluène puis la solution obtenue est concentrée à sec sous pression réduite (2,7 kPa) à 40°C . La même opération est répétée avec 40 cm3 de toluène. La meringue obtenue est dissoute dans 50 cm3 de dichlorométhane et la solution obtenue est additionnée de 25 cm3 d'une solution aqueuse saturée d'hydrogénocarbonate de sodium. La phase aqueuse est séparée par décantation et réextraite par 25 cm3 de dichlorométhane. Les phases organiques sont réunies, séchées sur sulfate de magnésium, filtrées puis concentrées à sec sous pression réduite (2,7 kPa) à 40°C. On obtient 0,65 g d'une meringue blanche que l'on purifie par chromatographie sur 40g de silice (O,063-0,2 mm) contenus dans une colonne de 2 cm de diamètre [éluant : dichlorométhane-méthanol (98-2 en volumes)] en recueillant des fractions de 20 cm3. Les fractions 5 à 9 sont réunies et concentrées à sec sous pression réduite (2,7 kPa) à une température voisine de 40°C. On obtient ainsi 0,45 g d'amino-3 hydroxy-2 (méthyl-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

Le tert-butoxycarbonyl-3 diméthyl-2,2 (méthyl-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α peut être préparé de la manière suivante :
A une solution de 0,4 g d'acide tert-butoxycarbonyl-3 diméthyl-2,2 (méthyl-4 phényl)-4 oxazolidinecarboxylique-5-(4S,5R) dans 10 cm3 de toluène on ajoute 0,247 g de N,N'-dicyclohexylcarbodiimide, 0,675 g d'acétoxy-4 benzoyloxy-2α époxy-5β,20 dihydroxy-1,13α oxo-9 bis-(trichloro-2,2,2 éthoxy) carbonyloxy-7β,10β taxène-11 et 0,046 g de diméthylamino-4 pyridine. Le milieu réactionnel est ensuite chauffé sous agitation pendant 3 heures à une température voisine de 80°C, puis refroidi à une température voisine de 20°C et additionné d'un mélange de 20 cm3 de dichlorométhane et de 25 cm3 d'une solution aqueuse saturée d'hydrogénocarbonate de sodium. La phase aqueuse est séparée par décantation puis réextraite par 15 cm3 de dichlorométhane. Les phases organiques sont réunies, séchées sur du sulfate de magnésium, filtrées puis concentrées à sec sous pression réduite (2,7 kPa) à 50°C. On obtient 1,1 g d'une meringue jaune que l'on purifie par chromatographie sur 40 g de silice (0,063-0,2 mm) contenus dans une colonne de 2 cm de diamètre [éluant : dichlorométhane-méthanol (98-2 en volumes)] en recueillant des fractions de 15 cm3. Les fractions 3 à 6 sont réunies et concentrées à sec sous pression réduite (2,7 kPa) à une température voisine de 40°C. On obtient ainsi 0,6 g de tert-butoxycarbonyl-3 diméthyl-2,2 (méthyl-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

L'acétoxy-4 benzoyloxy-2α époxy-5β,20 dihydroxy-1,13α oxo-9 bis-(trichloro-2,2,2 éthoxy) carbonyloxy-7β,10β taxène-11 peut être préparé selon la méthode décrite dans le brevet européen EP 0 336 841.

L'acide tert-butoxycarbonyl-3 diméthyl-2,2 (méthyl-4 phényl)-4 oxazolidinecarboxylique-5-(4S,5R) peut être préparé de la manière suivante :
A une solution de 0,54 g de tert-butoxycarbonyl-3 diméthyl-2,2 (méthyl-4 phényl)-4 oxazolidinecarboxylate-5-(4S,5R) d'éthyle dans 10 cm3 d'éthanol, on ajoute à une température voisine de 25°C, une solution de 0,19 g d'hydrate d'hydroxyde de lithium dans 3 cm3 d'eau distillée. Le milieu réactionnel est agité pendant 20 minutes à une température voisine de 25°C puis concentré à sec sous pression réduite (2,7 kPa) à une température voisine de 40°C. Le résidu obtenu est dissous dans 3,5 cm3 d'eau distillée puis extrait par 2 fois 1 cm3 d'oxyde de diisopropyle. La phase aqueuse est ensuite acidifiée à un pH voisin de 1 par 5 cm3 d'une solution aqueuse 1N d'acide chlorhydrique, puis extraite par 3 fois 4 cm3 de dichlorométhane. Les phases organiques sont réunies, séchées sur sulfate de magnésium, filtrées puis concentrées à sec sous pression réduite (2,7 kPa) à une température voisine de 40°C. On obtient ainsi 0,43 g d'acide tert-butoxycarbonyl-3 diméthyl-2,2 (méthyl-4 phényl)-4 oxazolidinecarboxylique-5-(4S,5R) sous forme d'une huile orange.

Le tert-butoxycarbonyl-3 diméthyl-2,2 (méthyl-4 phényl)-4 oxazolidinecarboxylate-5-(4S,5R) d'éthyle peut être préparé de la manière suivante :
Une solution de 0,63 g de tert-butoxycarbonylamino-3 hydroxy-2 (méthyl-4 phényl)-3 propionate-(2R,3S) d'éthyle, de 0,2 cm3 de méthoxy-2 propène et de 3,4 mg de p-toluène sulfonate de pyridinium dans 18 cm3 de toluène est agitée pendant 2 heures 30 minutes à une température voisine de 20°C. Le mélange réactionnel est chauffé jusqu'à l'ébullition et le distillat est recueilli dans un récipient gradué tandis que l'on additionne, goutte à goutte, une solution de 1,25 cm3 de méthoxy-2 propène dans 15 cm3 de toluène au milieu réactionnel de manière à maintenir constant le volume de ce milieu. Après 15 minutes de distillation, on ajoute 3,4 mg de p-toluènesulfonate de pyridinium puis la distillation est poursuivie pendant 15 minutes. Le volume de distillat recueilli est alors de 20 cm3. Le milieu réactionnel est refroidi à une température voisine de 20°C, on ajoute ensuite 2 cm3 d'une solution aqueuse saturée d'hydrogénocarbonate de sodium. La phase aqueuse est décantée, puis extraite par 2 fois 5 cm3 de dichlorométhane. Les phases organiques sont réunies, séchées sur sulfate de magnésium, filtrées et concentrées à sec sous pression réduite (2,3 kPa) à une température voisine de 40°C. On obtient ainsi 0,54 g de tert-butoxycarbonyl-3 diméthyl-2,2 (méthyl-4 phényl)-4 oxazolidinecarboxylate-5-(4S,5R) d'éthyle sous forme d'une huile jaune.

Le tert-butoxycarbonylamino-3 hydroxy-2 (méthyl-4 phényl)-3 propionate-(2R,3S) d'éthyle peut être préparé de la manière suivante :
A une solution de 0,8 g d'amino-3 hydroxy-2 (méthyl-4 phényl)-3 propionate-(2R,3S) d'éthyle dans 12 cm3 de dichlorométhane, maintenue sous atmosphère d'argon, on ajoute 0,33 g d'hydrogénocarbonate de sodium puis goutte à goutte, à une température voisine de 20°C, une solution de 0,94 g de dicarbonate de di.tert-butyle dans 4 cm3 de dichlorométhane. La solution obtenue est agitée pendant 2 heures 30 minutes à une température voisine de 20°C puis additionnée de 20 cm3 d'eau distillée. La phase aqueuse est séparée par décantation puis réextraite par 20 cm3 de dichlorométhane. Les phases organiques sont réunies, séchées sur sulfate de magnésium, filtrées puis concentrées à sec sous pression réduite (2,7 kPa) à 40°C. On obtient ainsi après cristallisation dans le cyclohexane 0,65 g de tert-butoxycarbonylamino-3 hydroxy-2 (méthyl-4 phényl)-3 propionate-(2R,3S) d'éthyle fondant à 130°C.

L'amino-3 hydroxy-2 (méthyl-4 phényl)-3 propionate- (2R,3S) d'éthyle peut être préparé de la manière suivante :
A une solution de 1,15 g d'azido-3 hydroxy-2 (méthyl-4 phényl)-3 propionate-(2R,3S) d'éthyle dans 35 cm3 d'acétate d'éthyle on ajoute 0,115 g de palladium à 10 % sur poudre de carbone. Le mélange réactionnel est agité sous une pression de 120 kPa d'hydrogène et à une température voisine de 22°C pendant 8 heures puis filtré sur verre fritté garni de célite. Le verre fritté est lavé par 5 cm3 d'acétate d'éthyle et les filtrats sont réunis puis concentrés à sec sous pression réduite (2,7 kPa) à une température voisine de 40°C. On obtient ainsi 0,83 g d'amino-3 hydroxy-2 (méthyl-4 phényl)-3 propionate-(2R,3S) d'éthyle sous forme d'une pâte jaune pâle.

L'azido-3 hydroxy-2 (méthyl-4 phényl)-3 propionate-(2R,3S) d'éthyle peut être préparé de la manière suivante :
A une solution de 2,2 g de (méthyl-4 phényl)-3 oxirannecarboxylate-2-(2R,3R) d'éthyle dans 60 cm3 d'éthanol on ajoute 1,04 g d'azoture de sodium et 0,86 g de chlorure d'ammonium. Le mélange réactionnel est agité à reflux pendant 5 heures 30 minutes puis refroidi à une température voisine de 20°C et additionné d'un mélange de 50 cm3 d'acétate d'éthyle et de 50 cm3 d'eau distillée. La phase aqueuse est séparée par décantation et réextraite par 50 cm3 d'acétate d'éthyle. Les phases organiques sont réunies, séchées sur sulfate de magnésium, filtrées puis concentrées à sec sous pression réduite (2,7 kPa) à 40°C. On obtient 2,4 g d'une huile orange que l'on purifie par chromatographie sur 80 g de silice (0,063-0,2 mm) contenus dans une colonne de 2 cm de diamètre [éluant : cyclohexane-acétate d'éthyle (90-10 en volumes)] en recueillant des fractions de 50 cm3. Les fractions 8 à 16 sont réunies et concentrées à sec sous pression réduite (2,7 kPa) à une température voisine de 40°C. On obtient ainsi 1,55 g d'azido-3 hydroxy-2 (méthyl-4 phényl)-3 propionate-(2R,3S) d'éthyle sous forme d'une huile orange.

Le (méthyl-4 phényl)-3 oxirannecarboxylate-(2R,3R) d'éthyle peut être préparé de la manière suivante :
A une solution, refroidie à une température voisine de -75°C, de 2,3 cm3 d'éthanol dans 40 cm3 de tétrahydrofuranne on ajoute en maintenant la température à -75°C, 25 cm3 d'une solution 1,6M de n-butyllithium dans l'hexane puis, goutte à goutte, une solution de 8,36 g de [bromo-2 hydroxy-3 (méthyl-4 phényl)-3 oxo-1 propyl]-3-(2S,3R) méthyl-4 phényl-5 oxazolidinone-2-(4S,5R) dans 120 cm3 de tétrahydrofuranne. Le milieu réactionnel est réchauffé jusqu'à une température voisine de 0°C puis maintenu à 0°C pendant 1 heure et refroidi à nouveau à une température voisine de -75°C. On ajoute ensuite en maintenant la température à -75°C, une solution de 5,04 g d'acide citrique dans 28 cm3 de tétrahydrofuranne. Le milieu réactionnel est réchauffé jusqu'à une température voisine de 15°C puis maintenu à 15°C pendant 1 heure et additionné d'un mélange de 40 cm3 d'eau distillée et de 200 cm3 d'éther diéthylique. La phase aqueuse est séparée par décantation et réextraite par 2 fois 50 cm3 d'éther diéthylique. Les phases organiques sont réunies, séchées sur sulfate de magnésium, filtrées puis concentrées à sec sous pression réduite (2,7 kPa) à 40°C. On obtient 5,3 g d'une huile orange que l'on purifie par chromatographie sur 200 g de silice (0,063-0,2 mm) contenus dans une colonne de 2cm de diamètre [éluant : cyclohexane-acétate d'éthyle (90-10 en volumes)] en recueillant des fractions de 50 cm3. Les fractions 10 à 20 sont réunies et concentrées à sec sous pression réduite (2,7 kPa) à une température voisine de 40°C. On obtient ainsi après cristallisation dans l'oxyde de diisopropyle 2,2 g de (méthyl-4 phényl)-3 oxirannecarboxylate-2-(2R,3R) d'éthyle fondant à 66°C.

Le [bromo-2 hydroxy-3 (méthyl-4 phényl)-3 oxo-1 propyl]-3-(2S,3R) méthyl-4 phényl-5 oxazolidinone-2-(4S,5R) peut être préparé de la manière suivante :
A une solution de 35,8 g de (bromo-2 oxo-1 éthyl)-3 méthyl-4 phényl-5 oxazolidinone-2-(4S,5R) dans 300 cm3 d'éther diéthylique anhydre, on ajoute à une température voisine de 20°C, 23,4 cm3 de triéthylamine puis, goutte à goutte, 135 cm3 d'une solution 1M de triflate de di-n-butylbore dans le dichlorométhane. Le milieu réactionnel est refroidi à une température voisine de -75°C puis on ajoute en maintenant la température à -75°C, 10,64 cm3 de méthyl-4 benzaldéhyde et réchauffe le milieu réactionnel jusqu'à une température voisine de 20°C et le maintient à 20°C pendant 18 heures. On ajoute ensuite 100 cm3 d'une solution saturée d'hydrogénosulfate de sodium et sépare la phase aqueuse par décantation et la réextrait par 2 fois 100 cm3 d'éther diéthylique. Les phases organiques sont réunies, séchées sur sulfate de magnésium, filtrées puis concentrées à sec sous pression réduite (2,7 kPa) à 40°C. On obtient 69 g d'une huile brune que l'on purifie par chromatographie sur 2000 g de silice (0,063-0,2 mm) contenus dans une colonne de 5 cm de diamètre [éluant : cyclohexane-acétate d'éthyle (70-30 en volumes)] en recueillant des fractions de 200 cm3. Les fractions 16 à 24 sont réunies et concentrées à sec sous pression réduite (2,7 kPa) à une température voisine de 40°C. On obtient ainsi après cristallisation dans l'oxyde de diisopropyle 17 g de [bromo-2 hydroxy-3 (méthyl-4 phényl)-3 oxo-1 propyl]-3-(2S,3R) méthyl-4 phényl-5 oxazolidinone-2-(4S,5R) fondant à 139°C.

La (bromo-2 oxo-1 éthyl)-3 méthyl-4 phényl-5 oxazolidinone-2-(4S,5R) peut être préparée de la manière suivante :
A une solution, refroidie à une température voisine de -75°C, de 106,2 g de méthyl-4 phényl-5 oxazolidinone-2-(4S,5R) dans 1080 cm3 de tétrahydrofuranne anhydre on ajoute en maintenant la température à -75°C, 375 cm3 d'une solution 1,6M de n-butyllithium dans l'hexane puis, goutte à goutte, 62,6 cm3 de bromure de bromacétyle. Le milieu réactionnel est agité à une température voisine de -70°C pendant 1 heure 30 minutes puis il est réchauffé jusqu'à une température voisine de -10°C et additionné de 600 cm3 d'une solution aqueuse saturée de chlorure d'ammonium. La phase aqueuse est séparée par décantation et réextraite par 500 cm3 d'éther diéthylique. Les phases organiques sont réunies, séchées sur sulfate de magnésium, filtrées puis concentrées à sec sous pression réduite (2,7 kPa) à 40°C. On obtient 201 g d'une huile jaune que l'on purifie par chromatographie sur 4000 g de silice (0,063-0,2 mm) contenus dans une colonne de 10 cm de diamètre [éluant : cyclohexane-acétate d'éthyle (66-33 en volumes)] en recueillant des fractions de 500 cm3. Les fractions 4 à 13 sont réunies et concentrées à sec sous pression réduite (2,7 kPa) à une température voisine de 40°C. On obtient ainsi 139,5 g de (bromo-2 oxo-1 éthyl)-3 méthyl-4 phényl-5 oxazolidinone-2-(4S,5R) sous forme d'une huile jaune pâle.

### EXEMPLE 2

En opérant comme dans l'exemple 1, mais à partir de tert-butoxycarbonylamino-3 (fluoro-2 phényl)-3 hydroxy-2 propionate-(2R,3S) d'acétoxy-4 benzoyloxy-2α époxy-5β,20 hydroxy-1 oxo-9 bis-(trichlor-2,2,2 éthoxy) carbonyloxy-7β,10β taxène-11 yle-13α, on obtient 0,17 g de tert-butoxycarbonylamino-3 (fluoro-2 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰D = -42° (c = 0,58 ; méthanol)
- spectre de RMN (400 MHz ; CDCl₃)
   δ (ppm) : 1,14 (s, 3H : -CH₃ 16 ou 17) ; 1,25 (s, 3H : -CH₃ 16 ou 17); 1,32 (s, 9H : -C(CH₃)₃) ; 1,76 (s, 3H : -CH₃ 19); 1,86 (m, 1H : -(CH)-H 6) ; 1,93 (s, 3H : -CH₃ 18) ; 2,22 (dd, 1H, J=9 et 16 Hz : -(CH)-H 14) ; 2,37 (dd, 1H, J=9 et 16 Hz : -(CH)-H 14) ; 2,45 (s, 3H : -COCH₃) ; 2,6 (m, 1H : -(CH)-H 6) ; 3,35 (s, 1H : -OH 2') ; 3,94 (d 1H, J=7 Hz : -H 3) ; 4,26 (AB, 2H, J_{AB}=9 Hz : -CH₂- 20) ; 4,28 (dd, 1H, J=7 et 12 Hz : -H 7) ; 4,62 (m, 1H : -H 2') ; 4,98 (d, 1H, J=9 Hz : -H 5) ; 5,23 (s, 1H : -H 10) ; 5,45 et 5,58 (d et d, 1H chacun, J=10 Hz : -CH-NHCO-) ; 5,7 (d, 1H, J=7 Hz : -H 2) ; 6,28 (t, 1H, J=9 Hz : -H 13) ; 7,06 à 7,4 (m, 4H : F-C₆H₄) ; 7,5 [t, 2H, J=8 Hz : -OCOC₆H₅ (-H 3 et -H 5)] ; 7,61 [t, 1H, J=8 Hz : -OCOC₆H₅ (-H 4)] ; 8,13 [d, 2H, J=8 Hz : -OCOC₆H₅ (-H 2 et -H 6)].

En opérant comme dans l'exemple 1, mais à partir de matières premières convenables, sont préparés les intermédiaires suivants :
- le tert-butoxycarbonylamino-3 (fluoro-2 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- l'amino-3 hydroxy-2 (fluoro-2 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- le tert-butoxycarbonyl-3 diméthyl-2,2 (fluoro-2 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- l'acide tert-butoxycarbonyl-3 diméthyl-2,2 (fluoro-2 phényl)-4 oxazolidinecarboxylique-5-(4S,5R) sous forme de cristaux blancs fondant à 164°C.
- le tert-butoxycarbonyl-3 diméthyl-2,2 (fluoro-2 phényl)-4 oxazolidinecarboxylate-5-(4S,5R) d'éthyle sous forme d'une huile jaune.
- le tert-butoxycarbonylamino-3 hydroxy-2 (fluoro-2 phényl)-3 propionate-(2R,3S) d'éthyle sous forme de cristaux blancs fondant à 99°C.
- l'amino-3 hydroxy-2 (fluoro-2 phényl)-3 propionate-(2R,3S) d'éthyle sous forme de cristaux blancs fondant à 73°C.
- l'azido-3 hydroxy-2 (fluoro-2 phényl)-3 propionate-(2R,3S) d'éthyle sous forme d'une huile jaune.
- le (fluoro-2 phényl)-3 oxirannecarboxylate-2-(2R,3R) d'éthyle sous forme d'une huile jaune.
- la [bromo-2 hydroxy-3 (fluoro-2 phényl)-3 oxo-1 propyl]-3-(2S,3R) méthyl-4 phényl-5 oxazolidinone-2-(4S,5R) sous forme d'une meringue jaune.

### EXEMPLE 3

En opérant comme dans l'exemple 1, mais à partir de tert-butoxycarbonylamino-3 (chloro-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α, on obtient 0,35 g de tert-butoxycarbonylamino-3 (chloro-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α²⁰D]= -27° (c = 0,97 ; méthanol)
- spectre de RMN (400 MHz ; CDCl₃)
   δ (ppm) : 1,15 (s, 3H : -CH₃ 16 ou 17) ; 1,25 (s, 3H : -CH₃ 16 ou 17) ; 1,35 (s, 9H : -C(CH₃)₃) ; 1,77 (s, 3H : -CH₃ 19) ; 1,9 (m, 1H : -(CH)-H 6 et s, 3H : -CH₃ 18) ; 2,3 (d, 2H, J=8,5 Hz : -CH₂ 14) ; 2,39 (s, 3H : -COCH₃) ; 2,6 (m, 1H : - (CH)-H 6) ; 3,48 (s, 1H : -OH 2') ; 3,92 (d, 1H, J=7 Hz : -H 3) ; 4,24 (dd, 1H, J=7 et 12 Hz : -H 7) ; 4,26 (AB, 2H, J_{AB}=9 Hz : -CH₂- 20) ; 4,61 (s, 1H : -H 2') ; 4,96 (d, 1H, J=9 Hz : -H 5) ; 5,24 (s, 1H : -H 10) ; 5,26 (m, 1H : -H 3') ; 5,43 (d, 1H, J=9 Hz : -NHCO-) ; 5,68 (d, 1H, J=7 Hz : -H 2) ; 6,25 (t, 1H, J=8,5 Hz : -H 13) ; 7,35 (m, 4H : Cl-C₆H₄) ; 7,5 [t, 2H, J=8 Hz : -OCOC₆H₅ (-H 3 et -H 5)] ; 7,62 [t, 1H, J=8 Hz : -OCOC₆H₅ (-H 4)] ; 8,10 [d, 2H, J=8 Hz : -OCOC₆H₅ (-H 2 et -H 6)].

En opérant comme dans l'exemple 1, mais à partir de matières premières convenables, sont préparés les intermédiaires suivants :
- le tert-butoxycarbonylamino-3 (chloro-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- l'amino-3 hydroxy-2 (chloro-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- le tert-butoxycarbonyl-3 diméthyl-2,2 (chloro-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- l'acide tert-butoxycarbonyl-3 diméthyl-2,2 (chloro-4 phényl)-4 oxazolidinecarboxylique-5-(4S,5R) sous forme d'une huile incolore.
- le tert-butoxycarbonyl-3 diméthyl-2,2 (chloro-4 phényl)-4 oxazolidinecarboxylate-5-(4S,5R) d'éthyle sous forme d'une huile jaune.
- le tert-butoxycarbonylamino-3 hydroxy-2 (chloro-4 phényl)-3 propionate-(2R,3S) d'éthyle sous forme de cristaux crème fondant à 117°C.
- l'amino-3 hydroxy-2 (chloro-4 phényl)-3 propionate-(2R,3S) d'éthyle sous forme d'une huile brune.
- l'azido-3 hydroxy-2 (chloro-4 phényl)-3 propionate-(2R,3S) d'éthyle sous forme d'une huile jaune.
- le (chloro-4 phényl)-3 oxirannecarboxylate-2-(2R,3R) d'éthyle sous forme d'une huile jaune.
- la [bromo-2 hydroxy-3 (chloro-4 phényl)-3 oxo-1 propyl]-3-(2S,3R) méthyl-4 phényl-5 oxazolidinone-2-(4S,5R) sous forme de cristaux blancs fondant à 140°C.

### EXEMPLE 4

En opérant comme dans l'exemple 1, mais à partir du tert-butoxycarbonylamino-3 (méthoxy-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α, on obtient 0,15 g de tert-butoxycarbonylamino-3 (méthoxy-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰D = -32° (c = 0,47; méthanol)
- spectre de RMN (400 MHz ; CDCl₃)
   δ (ppm) : 1,15 (s, 3H : -CH₃ 16 ou 17) ; 1,25 (s, 3H : -CH₃ 16 ou 17) ; 1,38 (s, 9H : -C(CH₃)₃) ; 1,7 (m, 1H : -OH 1) ; 1,78 (s, 3H : -CH₃ 19) ; 1,88 (m, 1H : -(CH)-H 6 et s, 3H : -CH₃ 18) ; 2,28 (d, 2H, J=8,5 Hz : -CH₂ 14) ; 2,38 (s, 3H : - COCH₃) ; 2,6 (m, 1H : -(CH)-H 6) ; 3,4 (m, 1H : -OH 2') ; 3,8 (s, 3H : -C₆H₄-OCH₃) ; 3,92 (d, 1H, J=7 Hz : -H 3) ; 4,2 et 4,33 (2d, 1H chacun, J=9 : Hz : -CH₂ 20) ; 4,25 (m, 1H : -H 7) ; 4,1 à 4,4 (m étalé, 1H : -OH 10) ; 4,59 (m, 1H : -H 2') ; 4,95 (d, 1H, J=9 Hz : -H 5) ; 5,2 et 5,37 (2m, 1H chacun : -CH-NHCOO-) ; 5,22 (s, 1H : -H 10) ; 5,69 (d, 1H, J=7 Hz : -H 2) ; 6,22 (t, 1H, J=8,5 Hz : -H 13) ; 6,92 [d, 2H, J=8 Hz : -C₆H₄-OCH₃ (-H 3 et -H 5)] ; 7,31 [d, 2H, J=8 Hz : -C₆H₄-OCH₃ (-H 2 et -H 6)] ; 7,45 [t, 2H, J=8 Hz : -OCOC₆H₅ (-H 3 et -H 5)] ; 7,62 [t, 1H, J=8 Hz : -OCOC₆H₅ (-H 4)] ; 8,11 [d, 2H, J=8 Hz : -OCOC₆H₅ (-H 2 et -H 6)].

En opérant comme dans l'exemple 1, mais à partir de matières premières convenables sont préparés les intermédiaires suivants :
- le tert-butoxycarbonylamino-3 (méthoxy-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- l'amino-3 hydroxy-2 (méthoxy-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- le tert-butoxycarbonylamino-3 diméthyl-2,2 (méthoxy-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- l'acide tert-butoxycarbonyl-3 diméthyl-2,2 (méthoxy-4 phényl)-4 oxazolidinecarboxylique-5-(4S,5R) sous forme d'une meringue blanche.
- le tert-butoxycarbonyl-3 diméthyl-2,2 (méthoxy-4 phényl)-4 oxazolidinecarboxylate-5-(4S,5R) d'éthyle sous forme d'une huile jaune.
- le tert-butoxycarbonylamino-3 hydroxy-2 (méthoxy-4 phényl)-3 propionate-(2R,3S) d'éthyle sous forme de cristaux blancs fondant à 135°C.
- l'amino-3 hydroxy-2 (méthoxy-4 phényl)-3 propionate-(2R,3S) d'éthyle sous forme d'une huile jaune.
- l'azido-3 hydroxy-2 (méthoxy-4 phényl)-3 propionate-(2R,3S) d'éthyle sous forme d'une huile jaune.
- le (méthoxy-4 phényl)-3 oxirannecarboxylate-2-(2R,3R) d'éthyle sous forme d'une huile jaune.
- la [bromo-2 hydroxy-3 (méthoxy-4 phényl)-3 oxo-1 propyl]-3-(2S,3R) méthyl-4 phényl-5 oxazolidinone-2-(4S,5R) sous forme de cristaux blancs fondant à 130°C.

### EXEMPLE 5

En opérant comme à l'exemple 1, mais à partir du tert-butoxycarbonylamino-3 (fluoro-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α, on obtient 0,086 g de tert-butoxycarbonylamino-3 (fluoro-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰D = -35° (c = 0,49 ; méthanol)
- spectre de RMN (250 MHz ; CDCl₃)
   (δ ppm) : 1,14 (s, 3H : -CH₃ 16 ou 17) ; 1,25 (s, 3H : -CH₃ 16 ou 17) ; 1,35 (s, 9H : -C(CH₃)₃) ; 1,7 (m, 1H : -OH 1); 1,77 (s, 3H : -CH₃ 19) ; 1,87 (m, 1H : -(CH)-H 6) ; 1,87 (s, 3H :-CH₃ 18) ; 2,3 (d, 2H, J=9Hz : -C-H₂14) ; 2,36 (s, 3H : -COCH₃) ; 2,6 (m, 1H : -(CH)-H 6) ; 3,43 (m, 1H : -OH 2') ; 3,93 (d, 1H, J=7 Hz : -H 3) ; 4,2 et 4,33 (AB, 2H, J_{A-B}=8 Hz : -CH₂ 20) ; 4,23 (m, 1H : -H 7) ; 4,6 (m, 1H : -H 2') ; 4,96 (dd, 1H, J=2 et 10,5 Hz : -H 5) ; 5,22 (s, 1H : -H 10) ; 5,25 (m, 1H : -H 3') ; 5,42 (d, 1H, J=10 Hz : -CH-NHCO-) ; 5,7 (dd, 1H, J=7 Hz : -H 2) ; 6,24 (t, 1H, J=9 Hz : -H 13) ; 7,09 [t, 2H, J=8,5 Hz : F-C₆H₄ (-H 3 et -H 5)] ; 7,38 [dd, 2H, J=8,5 Hz : F-C₆H₄ (-H 2 et -H 6)] ; 7,5 [t, 2H, J=8,5 Hz : -OCOC₆H₅ (-H 3 et -H 5)] ; 7,62 [t, 1H, J=8,5 Hz : -OCOC₆H₅ (-H 4)] ; 8,1 [d, 2H, J=8,5 Hz : - OCOC₆H₅ (-H 2 et -H 6)].

En opérant comme dans l'exemple 1, mais à partir de matières premières convenables, sont préparés les intermédiaires suivants :
- le tert-butoxycarbonylamino-3 (fluoro-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche,
- l'amino-3 hydroxy-2 (fluoro-4 phényl)-3 propionate- (2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche,
- le tert-butoxycarbonyl-3 diméthyl-2,2 (fluoro-4 phényl)-4 oxazolidine carboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche,
- l'acide tert-butoxycarbonyl-3 diméthyl-2,2 (fluoro-4 phényl)-4 oxazolidinecarboxylique-5-(4S,5R) sous forme d'une huile jaune,
- le tert-butoxycarbonyl-3 diméthyl-2,2 (fluoro-4 phényl)-4 oxazolidinecarboxylate-5-(4S,5R) d'éthyle sous forme d'une huile jaune,
- le tert-butoxycarbonylamino-3 hydroxy-2 (fluoro-4 phényl)-3 propionate-(2R,3S) d'éthyle sous forme de cristaux blancs fondant à 116°C,
- l'amino-3 hydroxy-2 (fluoro-4 phényl)-3 propionate-(2R,3S) d'éthyle sous forme de cristaux blancs fondant à 105°C,
- l'azido-3 hydroxy-2 (fluoro-4 phényl)-3 propionate- (2R,3S) d'éthyle sous forme d'une huile jaune,
- le (fluoro-4 phényl)-3 oxirannecarboxylate-2-(2R,3S) d'éthyle sous forme de cristaux jaune pâle fondant à 40°C,
- le [bromo-2 hydroxy-3 (fluoro-4 phényl)-3 oxo-1 propyl]-3-(2S,3R) méthyl-4 phényl-5 oxazolidinone-2-(4S,5R) sous forme d'une meringue jaune.

Les nouveaux produits de formule générale (I) présentent des activités biologiques particulièrement intéressantes.

Les nouveaux produits de formule générale (Ia) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (Ia) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérélisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons(α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomusine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées tel que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogène comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et , encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par introduction dans une perfusion d'un soluté physiologique pendant 1 heure.

## Revendications

1. Procédé de préparation de dérivés du taxane de formule générale : 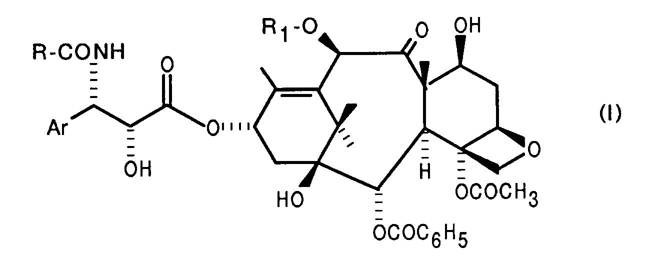 dans laquelle R représente un radical t.butoxy ou phényle, R₁ représente un atome d'hydrogène et Ar représente un radical phényle substitué ou un radical α- ou β-naphtyle éventuellement substitué par un ou plusieurs atomes ou radicaux choisis parmi les atomes d'halogène (fluor, chlore, brome, iode) et les radicaux alcoyles, aryles, arylalcoyles, alkoxy, alkylthio, aryloxy, arylthio, mercapto, acylamino, aroylamino, alkoxycarbonylamino, amino, alkylamino, dialkylamino, carboxy, alkoxycarbonyle, carbamoyle, dialkylcarbamoyle, cyano, nitro et trifluorométhyle, étant entendu que les radicaux alkyles et les portions alkyles des autres radicaux contiennent 1 à 4 atomes de carbone et que les radicaux aryles sont des radicaux phényles ou α- ou β-naphtyles, caractérisé en ce que :
a) on condense un dérivé de l'oxazolidine de formule générale : 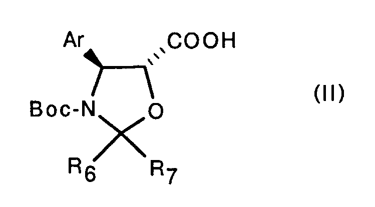 dans laquelle Ar est défini comme précédemment, Boc représente le radical t.butoxycarbonyle et R₆ et R₇, identiques ou différents, représentent un radical alkyle contenant 1 à 4 atomes de carbone éventuellement substitué par un ou plusieurs radicaux aryles (phényle), ou aryle (phényle), ou bien R₆ et R₇ forment ensemble avec l'atome de carbone auquel ils sont liés un cycle ayant de 4 à 7 chaînons, sur un dérivé du taxane de formule générale : 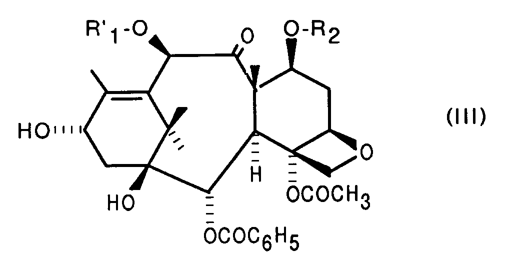 dans laquelle R'₁ représente un radical acétyle ou un groupement protecteur de la fonction hydroxy et R₂ représente un groupement protecteur de la fonction hydroxy, pour obtenir un produit de formule générale : 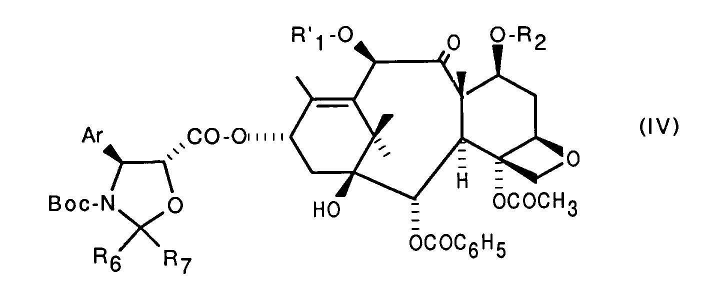 dans laquelle Ar, R'₁, R₂, R₆ et R₇ sont définis comme précédemment,
b) on traite en milieu acide, dans des conditions qui sont sans effet sur R'₁ et R₂, pour obtenir le produit de formule générale : 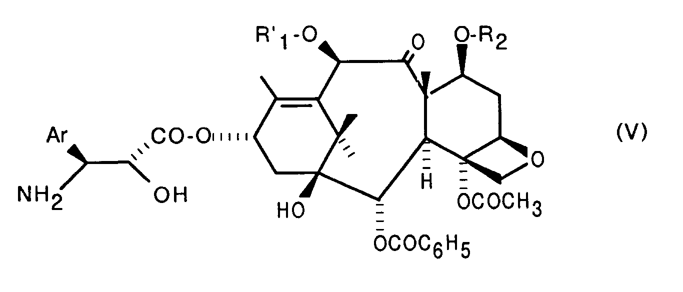 dans laquelle R'₁ et R₂ sont définis comme precédemment,
c) on traite par un réactif convenable permettant d'introduire un radical t.butoxycarbonyle ou benzoyle sur la fonction amino, pour obtenir un produit de formule générale : 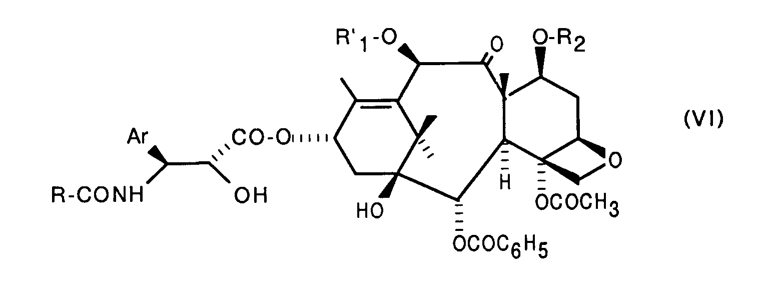
d) on remplace les groupements protecteurs R'₁ et R₂ par des atomes d'hydrogène dans des conditions appropriées puis,
e) on isole le produit obtenu et éventuellement le purifie.

2. Procédé selon la revendication 1 caractérisé en ce que les radicaux protecteurs représentés par R'₁ et R₂ sont des radicaux trichloro-2,2,2 éthoxycarbonyle ou des radicaux pouvant être transformés en radicaux trichloro-2,2,2 éthoxycarbonyle.

3. Procédé selon l'une des revendications 1 ou 2 caractérisé en ce que la condensation du produit de formule générale (II) sur le dérivé du taxane de formule générale (III) est effectuée en présence d'un agent de condensation et d'un agent d'activation.

4. Procédé selon l'une des revendications 1 à 3 caractérisé en ce que le traitement sélectif du produit de formule générale (IV) est effectué au moyen d'un acide minéral ou organique éventuellement dans un alcool.

5. Procédé selon l'une des revendications 1 à 4 caractérisé en ce que l'introduction d'un radical t.butoxycarbonyle ou benzoyle sur le produit de formule générale (V) est effectuée au moyen de dicarbonate de di-t.butyle ou de chlorure de benzoyle.

6. Procédé selon l'une des revendications 1 à 5 caractérisé en ce que le remplacement des groupements protecteurs représentés par R'₁ et R₂ par des atomes d'hydrogène est effectué par le zinc en milieu acide acétique ou au moyen d'un acide minéral ou organique dans un alcool aliphatique en présence de zinc.

7. Les nouveaux dérivés du taxane de formule générale : 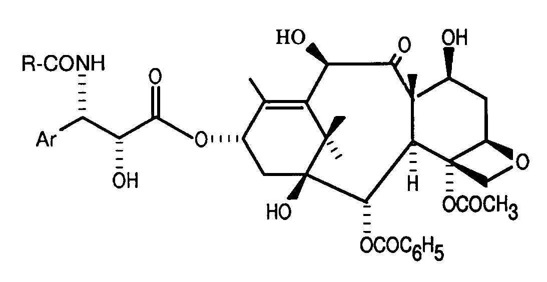 dans laquelle R représente un radical t.butoxy ou phényle et Ar représente un radical phényle substitué ou α- ou β-naphtyle éventuellement substitué par un ou plusieurs atomes ou radicaux choisis parmi les atomes d'halogène (fluor, chlore, brome, iode) et les radicaux alkyles,aryles, aralkyles, alkoxy, alkylthio, aryloxy, arylthio, mercapto, acylamino, aroylamino, alkoxycarbonylamino, amino, alkylamino, dialkylamino, carboxy, alkoxycarbonyle, carbamoyle, dialkylcarbamoyle, cyano, nitro et trifluorométhyle, étant entendu que les radicaux alkyles et les portions alkyles des autres radicaux contiennent 1 à 4 atomes de carbone et que les radicaux aryles sont des radicaux phényles ou α- ou β-naphtyles.

8. Composition pharmaceutique caractérisée en ce quelle contient une quantité suffisante d'au moins un dérivé selon la revendication 7 à l'état pur ou en association avec un ou plusieurs produits pharmaceutiquement acceptables qu'ils soient inertes ou physiologiquement actifs.

9. Utilisation d'un produit selon la revendication 7 pour la préparation d'un médicament pour le traitement des désordres dus à la prolifération anormale des cellules chez l'homme et les mammifères.

10. Les dérivés de l'oxazolidine de formule générale : 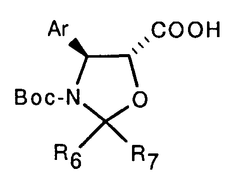 dans laquelle Ar est défini selon la revendication 1, Boc représente le radical t.butoxycarbenyle et R₆ et R₇, identiques ou différents, représentent un radical alkyle contenant 1 à 4 atomes de carbone éventuellement substitué par un ou plusieurs radicaux aryles (phényle) ou aryle (phényle), ou bien R₆ et R₇ forment ensemble avec l'atome de carbone auquel ils sont liés un cycle ayant de 4 à 7 chaînons.

11. Procédé de préparation d'un dérivé du l'oxazolidine selon la revendication 10 caractérisé en ce que l'on fait réagir un méthoxyalcène éventuellement substitué par un ou plusieurs radicaux aryles, un diméthoxyalcane éventuellement substitué par un ou plusieurs radicaux aryles ou un gem-diméthoxycycloalcane contenant 4 à 7 atomes de carbone sur un dérivé de la phénylisosérine de formule générale : 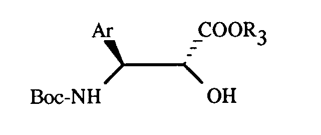 dans laquelle Ar est défini selon l'une des revendications 1 ou 2, Boc représente le radical t.butoxycarbonyle et R₃ représente un radical alkyle contenant 1 à 4 atomes de carbone éventuellement substitué par un radical phényle, puis saponifie le produit obtenu.

## Claims

1. Process for the preparation of taxane derivatives of general formula: 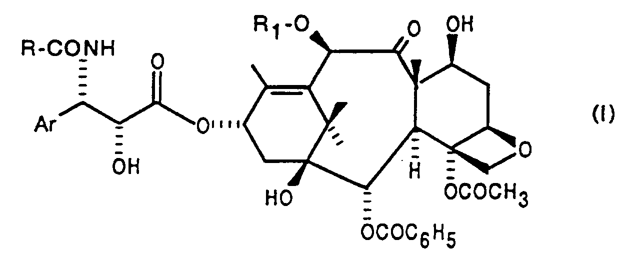 in which R represents a t-butoxy or phenyl radical, R₁ represents a hydrogen atom and Ar represents a substituted phenyl radical or an α- or β-naphthyl radical which is optionally substituted with one or more atoms or radicals chosen from halogen atoms (fluorine, chlorine, bromine or iodine) and alkyl, aryl, arylalkyl, alkoxy, alkylthio, aryloxy, arylthio, mercapto, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms and that the aryl radicals are phenyl or α-or β-naphthyl radicals, characterized in that:
a) an oxazolidine derivative of general formula: 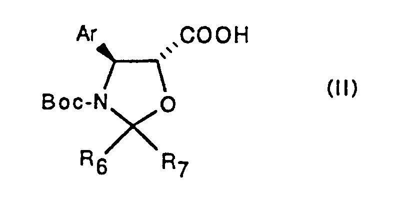 in which Ar is defined as above, Boc represents the t-butoxycarbonyl radical and R₆ and R₇, which are identical or different, represent an alkyl radical containing 1 to 4 carbon atoms which is optionally substituted with one or more aryl (phenyl) radicals, or an aryl (phenyl) radical, or alternatively R₆ and R₇ form, together with the carbon atom to which they are attached, a 4- to 7-membered ring, is condensed with a taxane derivative of general formula: 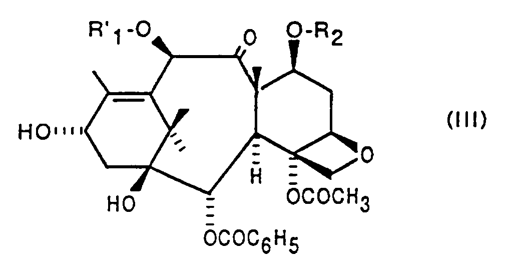 in which R'₁ represents an acetyl radical or a protecting group for the hydroxyl function and R₂ represents a protecting group for the hydroxyl function, in order to obtain a product of general formula: 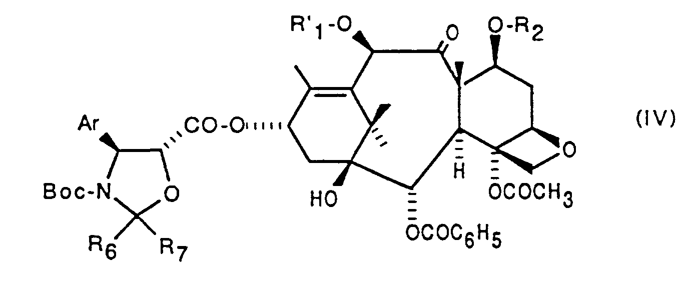 in which Ar, R'₁, R₂, R₆ and R₇ are defined as above,
b) treatment is carried out in acidic medium, under conditions which have no effect on R'₁ and R₂, in order to obtain the product of general formula: 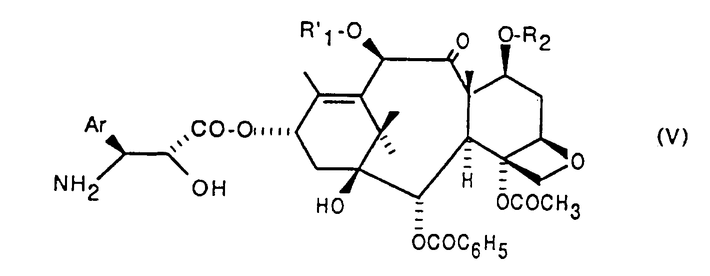 in which R'₁ and R₂ are defined as above,
c) treatment is carried out with a suitable reactant which allows the introduction of a t-butoxycarbonyl or benzoyl radical onto the amino function, in order to obtain a product of general formula: 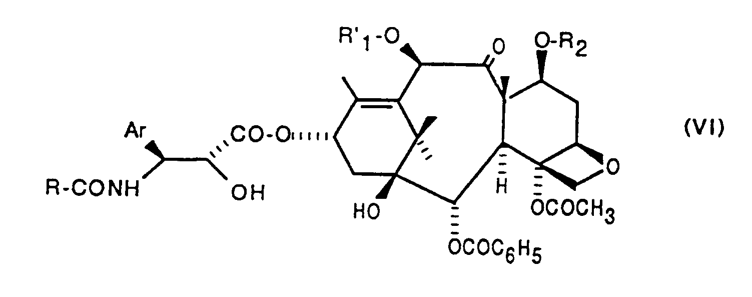
d) the protecting groups R'₁ and R₂ are replaced with hydrogen atoms under suitable conditions, and
e) the product obtained is then isolated and optionally purified.

2. Process according to claim 1, characterized in that the protecting radicals represented by R'₁ and R₂ are 2,2,2-trichloroethoxycarbonyl radicals or radicals which may be converted to 2,2,2-trichloroethoxycarbonyl radicals.

3. Process according to either of claims 1 and 2, characterized in that condensation of the product of general formula (II) with the taxane derivative of general formula (III) is carried out in the presence of a coupling agent and of an activating agent.

4. Process according to one of claims 1 to 3, characterized in that the selective treatment of the product of general formula (IV) is carried out using an inorganic or organic acid, optionally in an alcohol.

5. Process according to one of claims 1 to 4, characterized in that introduction of a t-butoxycarbonyl or benzoyl radical into the product of general formula (V) is carried out using di-t-butyl dicarbonate or benzoyl chloride.

6. Process according to one of claims 1 to 5, characterized in that replacement of the protecting groups represented by R'₁ and R₂ with hydrogen atoms is carried out by zinc in acetic acid medium or using an inorganic or organic acid in an aliphatic alcohol in the presence of zinc.

7. The new taxane derivatives of general formula: 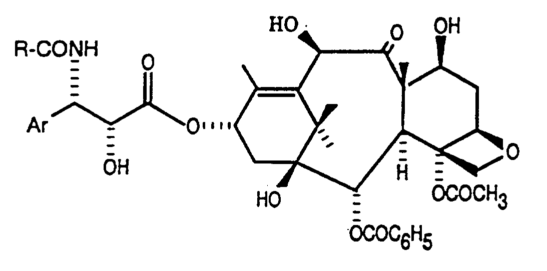 in which R represents a t-butoxy or phenyl radical and Ar represents a substituted phenyl or α- or β-naphthyl radical which is optionally substituted with one or more atoms or radicals chosen from halogen atoms (fluorine, chlorine, bromine or iodine) and alkyl, aryl, aralkyl, alkoxy, alkylthio, aryloxy, arylthio, mercapto, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms and that the aryl radicals are phenyl or α- or β-naphthyl radicals.

8. Pharmaceutical composition, characterized in that it contains a sufficient quantity of at least one derivative according to claim 7 in the pure state or combined with one or more pharmaceutically acceptable products, whether they are inert or physiologically active.

9. Use of a product according to claim 7 for the preparation of a medicament for the treatment of disorders due to the abnormal proliferation of cells in man and mammals.

10. The oxazolidine derivatives of general formula: 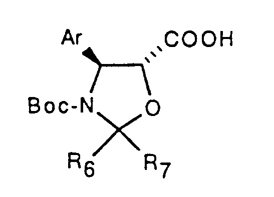 in which Ar is defined according to claim 1, Boc represents the t-butoxycarbonyl radical and R₆ and R₇, which are identical or different, represent an alkyl radical containing 1 to 4 carbon atoms which is optionally substituted with one or more aryl (phenyl) radicals, or an aryl (phenyl) radical, or alternatively R₆ and R₇ form, together with the carbon atom to which they are attached, a 4- to 7-membered ring.

11. Process for the preparation of an oxazolidine derivative according to claim 10, characterized in that a methoxyalkene which is optionally substituted with one or more aryl radicals, a dimethoxyalkane which is optionally substituted with one or more aryl radicals or a gemdimethoxycycloalkane containing 4 to 7 carbon atoms is reacted with a phenylisoserine derivative of general formula: 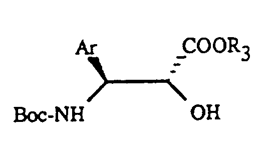 in which Ar is defined according to either of claims 1 and 2, Boc represents the t-butoxycarbonyl radical and R₃ represents an alkyl radical containing 1 to 4 carbon atoms which is optionally substituted with a phenyl radical, and the product obtained is then saponified.

## Patentansprüche

1. Verfahren zur Herstellung von Taxanderivaten mit der allgemeinen Formel: 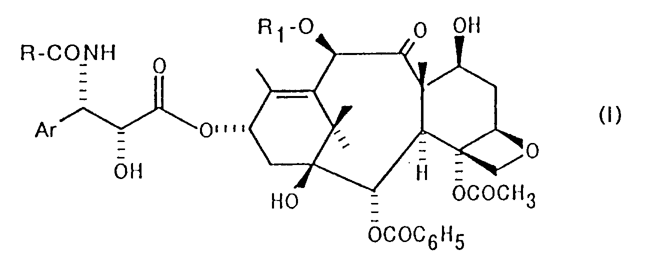 in der R für einen tert-Butoxy- oder Phenylrest sieht, R₁ für ein Wasserstoffatom und Ar für einen substituierten Phenylrest oder einen (α- oder β-Naphthylrest, der gegebenenfalls mit einen, oder mehreren Atomen oder Resten substituiert ist, ausgewählt aus den Halogenatomen (Fluor, Chlor, Brom, Iod) und den Alkyl-, Aryl-, Arylalkyl-, Alkoxy-, Alkylthio-,Aryloxy-, Arylthio-, Mercapto-, Acylamino-, Aroylamino-, Alkoxycarbonylamino-, Amino-, Alkylamino-, Dialkylamino-, Carboxy-, Alkoxycarbonyl-, Carbamoyl-, Dialkylcarbamoyl-, Cyano-, Nitro- und Trifluormethylresten, wobei die Alkylreste und die Alkylanteile der anderen Reste 1 bis 4 Kohlenstoffatome enthalten und die Arylreste Phenyl- oder α-oder β-Naphthylreste sind,dadurch gekennzeichnet,
a) daß man ein Oxazolidinderivat mit der allgemeinen Formel: 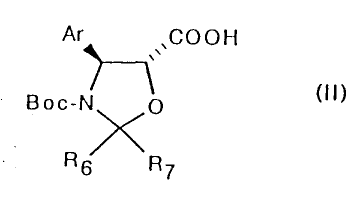 in der Ar wie vorher definiert ist, Boc den tert-Butoxycarbonylrest darstellt und R₆ und R₇, gleich oder verschieden, für einen Alkylrest mit 1 bis 4 Kohlenstoffatomen, der gegebenenfalls mit einen, Arylrest (Phenylrest) oder mehreren Arylresten (Phenylresten) substituiert ist, oder für Aryl (Phenyl) stehen oder aber R₆ und R₇ zusammen mit den, Kohlenstoffatom, an das sie gebunden sind, einen Ring mit 4 bis 7 Gliedern Bilden, mit einen, Taxan mit folgender allgemeiner Formel kondensiert: 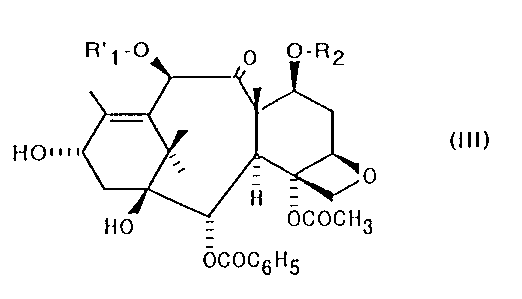 in der R'₁ für einen Acetylrest oder eine Schutzgruppe für die Hydroxylfunktion steht und R₂ für eine Schutzgruppe für die Hydroxylfunktion, wodurch man ein Produkt mit folgender allgemeiner Formel erhält: 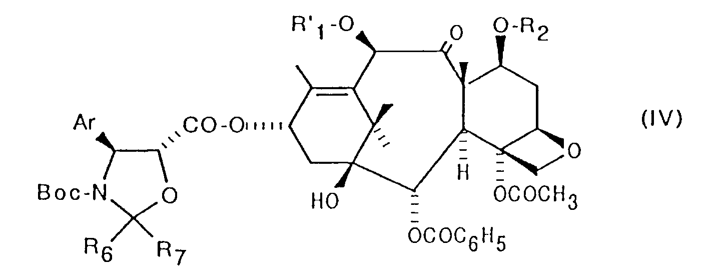 in der Ar, R'₁, R₂, R₆ und R₇ wie vorher definiert sind,
b) daß man dieses Produkt in saurem Milieu behandelt unter Bedingungen, die keine Wirkung auf R'₁ und R₂ ausüben, wodurch man das Produkt mit folgender allgemeiner Formel erhält: 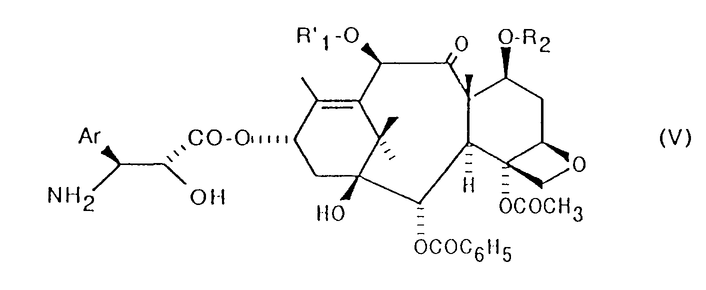 in der R'₁ und R₂ wie vorher definiert sind,
c) daß mal, dieses Produkt mit einem passenden Reagenz zum Einbau eines tert-Butoxycarbonyl- oder Benzoylrests in die Aminofunktion behandelt, wodurch man ein Produkt mit folgender allgemeiner Formel erhält: 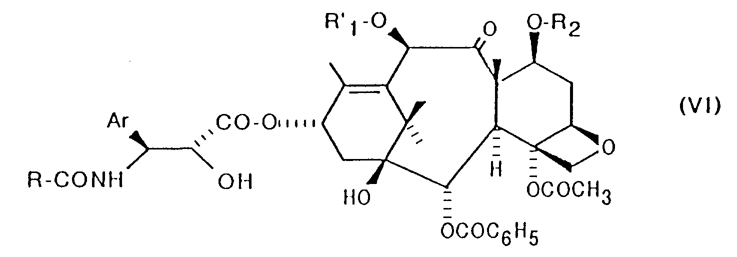
d) daß man die Schutzgruppen R'₁ und R₂ unter geeigneten Bedingungen durch Wasserstoffatome ersetzt,
e) daß man dann das erhaltene Produkt isoliert und es gegebenenfalls reinigt.

2. Verfahren nach Anspruch 1, dadurch gekennzeichnet, daß die Schutzgruppen, die von R'₁ und R₂ dargestellt werden, 2,2,2-Trichlorethoxycarbonylreste sind oder Reste, die in 2,2,2-Trichlorethoxycarbonylreste umgewandelt werden können.

3. Verfahren nach einem der Ansprüche 1 oder 2, dadurch gekennzeichnet, daß die Kondensation des Produktes mit der allgemeinen Formel (II) mit einem Taxanderivat mit der allgemeinen Formel (III) in Gegenwart eines Kondensationsmittels und eines Aktivators durchgeführt wird.

4. Verfahren nach einem der Ansprüche 1 bis 3, dadurch gekennzeichnet, daß die selektive Behandlung des Produkts mit der allgemeinen Formel (IV) mit einer Mineralsäure oder einer organischen Säure gegebenenfalls in einem Alkohol durchgeführt wird.

5. Verfahren nach einem der Ansprüche 1 bis 4, dadurch gekennzeichent, daß der Einbau eines tert-Butoxycarbonyl- oder Benzoylrests in das Produkt mit der allgemeinen Formel (V) mit Di-tert-butyldicarbonat oder Benzoylchlorid durchgeführt wird.

6. Verfahren nach einem der Ansprüche 1 bis 5, dadurch gekennzeichnet, daß das Ersetzen der Schutzgruppen, die von R'₁ und R₂ dargestellt werden, durch Wasserstoffatome mit Zink im essigsaurem Milieu durchgeführt wird oder mit einer Mineralsäure oder organischen Säure in einem aliphatischen Alkohol in Gegenwart von Zink.

7. Die neuen Taxanderivate mit der allgemeinen Formel: 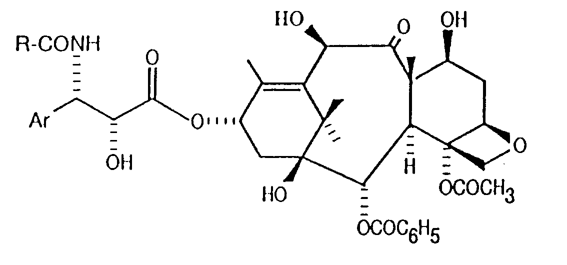 in der R für einen tert-Butoxycarbonyl- oder Phenylrest steht und Ar für einen substituierten Phenylrest oder einen α-oder β-Naphthylrest, der gegebenenfalls mit einem oder mehreren Atomen oder Resten substituiert ist, ausgewählt aus den Halogenatomen (Fluor, Chlor, Brom, Iod) und den Alkyl-, Aryl-, Aralkyl-, Alkoxy- Alkylthio, Aryloxy-, Arylthio, Mercapto-, Acylamino-, Aroylamino-, Alkoxycarbonylamino-, Amino-, Alkylamino-, Dialkylamino-, Carboxy-, Alkoxycarbonyl-, Carbamoyl-, Dialkylcarbamoyl-, Cyano-, Nitro- und Trifluormethylresten, wobei die Alkylreste und die Alkylanteile der anderen Reste 1 bis 4 Kohlenstoffatome enthalten und die Arylreste Phenylreste oder α- oder β-Naphthylreste sind.

8. Pharmazeutische Zusammensetzung, dadurch gekennzeichnet, daß sie eine ausreichende Menge zumindest eines Derivates nach Anspruch 7 in reinem Zustand oder in Verbinding mit einem oder mehreren pharmazeutisch verwendbaren Produkten enthält, die inert oder physiologisch aktiv sein können.

9. Verwendung eines Produkts nach Anspruch 7 zur Herstellung eines Arzneimittels zur Behandlung von Störungen, die auf der anormalen Zellproliferation beim Menschen oder den Säugetieren beruhen.

10. Die Oxazolidinderivate mit der allgemeinen Formel: 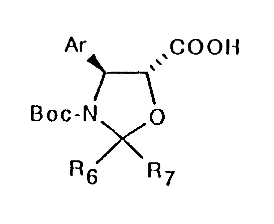 In der Ar wie in Anspruch 1 definiert ist, Boc den tert-Butoxycarbonylrest darstellt und R₆ und R₇, gleich oder verschieden, für einen Alkylrest mit 1 bis 4 Kohlenstoffatomen, der gegebenenfalls mit einem Arylrest (Phenylrest) oder mehreren Arylresten (Phenylresten) substituiert ist, oder für Aryl (Phenyl) stehen oder aber R₆ und R₇ zusammen mit dem Kohlenstoffatom, an das sie gebunden sind, einen Ring mit 4 bis 7 Gliedern bilden.

11. Verfahren zur Herstellung eines Oxazolidinderivates nach Anspruch 10, dadurch gekennzeichnet, daß man ein Methoxyalken, das gegebenenfalls mit einem oder mehreren Arylresten substituiert ist, ein Dimethoxyalkan, das gegebenenfalls mit einem oder mehreren Arylresten substituiert ist oder ein gem-Dimethoxycycloalkan mit 4 bis 7 Kohlenstoffatomen mit einem Phenylisoserinderivat mit folgender allgemeiner Formel reagieren läßt: 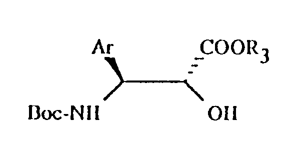 In der Ar gemäß einem der Ansprüche 1 oder 2 definiert ist, Boc für den tert-Butoxycarbonylrest steht und R₃ für einen Alkylrest, der 1 bis 4 Kohlenstoffatome enthält und der gegebenenfalls mit einem Phenylrest substituiert ist, daß man dann das erhaltene Produkt verseift.
